# EUROPEAN PATENT APPLICATION

(11) **EP 1 895 819 A1**
(43) Date of publication of application: **05.03.2008**
(21) Application number: 06119730.7
(22) Date of filing: 29.08.2006
(51) Int. Cl.: H05H 6/00, G21G 1/10, G21G 4/02

(54) **Neutron generating device for boron neutron capture therapy**

(71) Applicant: ION BEAM APPLICATIONS S.A., 1348 Louvain-la-Neuve (BE)
(72) Inventor: Jongen, Yves, 1348 Louvain-la-Neuve (BE); Stichelbaut, Frédéric, 5032 Mazy (BE); Cambriani, Andrea, 1050 Bruxelles (BE); Lucas, Stéphane, 5020 Suarlée (BE); Bodart, Franz, 5004 Bouge (BE); Burdakov, Alexander, 630117 Novosibirsk (RU)
(74) Representative: pronovem

(57) **Abstract**

The present invention is related to a target (1) for producing neutrons from a 20 mA, 1.9 to 3 MeV proton beam, the target comprising a thin layer (16) of lithium arranged inclined with reference to the incident proton beam. The lithium layer is provided on a backing (15) made of a material having as one of its main constituents an element chosen out of the group comprising Fe, Ta and V. The target is preferably comprised of two panels arranged at an angle with respect to each other. The backing preferably comprises cooling means. The beam shaping assembly for the target preferably comprises a moderator made of magnesium fluoride.

## Description

### Field of the Invention

The present invention is related to a neutron generating device. More particularly, the invention relates to a neutron generating device suitable for boron neutron capture therapy.

### State of the Art

Neutron beams are used in radiotherapy as means for treating cancer. One particular cancer therapy is the boron neutron capture therapy (BNCT). Clinical interest in BNCT has focused primarily on the treatment of gliomas. Only recently the treatment of other types of cancers such as melanomas and head, neck and liver cancer are being investigated.

BNCT provides a way to selectively destroy malignant cancer cells, while sparing the healthy ones. It is based on the nuclear capture and fission reactions that occur when boron-10 (¹⁰B) is irradiated with epithermal neutrons to yield a compound nucleus (excited boron-11) which then promptly disintegrates into lithium-7 (⁷Li) and an alpha particle. Both the alpha particle and the lithium ion produce closely spaced ionizations in the immediate vicinity of the reaction. As the particles have a limited path length of approximately 10 µm, which corresponds to the diameter of a cell, the destructive effects of the therapy are limited to the cells containing boron.

Boron is administered to the patient through a boron delivery agent. Various different boron-containing compounds have been designed and synthesized for BNCT, among others sodium borocaptate (Na₂B₁₂H₁₁SH), boronphenylalanine and D,L-4-(2-carboxyl-2-aminethyl) benzene boronic acid. The requirement for selective tumour targeting is to achieve boron concentrations sufficient to deliver therapeutic doses of radiation to the tumour with minimal normal tissue toxicity. A boron concentration of about 20µg ¹⁰B/g in the tumour is generally regarded as a sufficient therapeutic dose. Combinations of delivery agents are also used. See also R.F. Barth et al., "Boron neutron capture therapy of cancer: current status and future prospects" in Clinical Cancer Research, Vol. 11 (2005), pp. 3987-4002.

Preferably epithermal neutrons are used in BNCT. A pure thermal neutron beam leads to an exponential attenuation of the neutron fluence, which makes it difficult to reach deeply seated tumours. Epithermal neutrons, having an energy between 0.1 eV and 20 keV allow to obtain a maximal neutron fluence at a few cm inside the tissue, giving the possibility to treat deeply seated tumours.

Neutron sources for boron neutron capture therapy may be nuclear reactors or charged particle accelerators. Accelerators have the potential to produce a neutron beam with an energy distribution that is better than that of a reactor, i.e. lower and less widely distributed neutron energies. Accelerators are also more compact than reactors and better suited to be hosted in hospitals.

An accelerator produces a proton or deuteron beam having a specified energy. The proton or deuteron beam is used in a nuclear reaction to yield a neutron beam with specified energy and energy distribution useful for BNCT (epithermal neutrons). The protons that are output by the accelerator are directed to a target. Bombardment of the target material by the protons causes a nuclear reaction which generates neutrons.

The neutrons generated in the target generally have a too high energy to be suitable for BNCT. A beam shaping assembly is therefore necessary in order to reduce the energy of the neutrons to the epithermal range. At the heart of such beam shaping assembly lies the moderator, which provides the specified neutron flux. The moderator also attenuates the neutrons to the epithermal energy range.

The moderator may be surrounded by a reflector, a delimiter and a filter. Used moderator materials may be MgF₂, D₂O, Fluental® (compound made of aluminium fluoride, lithium fluoride and aluminium, such as disclosed in patent US 5703918) and polytetrafluoroethylene. Different moderator materials are discussed by Darren Leo Bleuel in his dissertation "Determination and Production of an Optimal Neutron Energy Spectrum for Boron Neutron Capture Therapy" (University of California at Berkeley, 2003). In this dissertation, many materials were considered as moderator: heavy water (D₂O); berillya (BeO); Fluental; enriched ⁷LiF; Teflon (CF₂); Iron. The author also discusses the properties of lithium used as a target. A thin lithium layer (45 µm) mounted on a metallic backing of a material such as copper (Cu) or aluminium (Al) is disclosed.

Furthermore, the epithermal neutrons directed to the patient must be collimated. This may be performed before or after the moderator and before or after the target.

Lithium is a widely used target material because of its high yield. Bombardment of lithium by a 10-20 mA, 1.9-3 MeV proton beam leads to the reaction ⁷i(p,n)⁷Be, where p stands for protons and n for neutrons. Hence, upon irradiation with protons, lithium is transmuted into beryllium and high energy neutrons are produced. The ⁷Li(p,n)⁷Be reaction occurs only upon bombardment with protons above the 1.889 MeV threshold. Protons of smaller energy do not participate in the generation of neutrons.

As the proton beam strikes the target material, it loses intensity. The generation of neutrons occurs for proton beams of energy above 1.889 MeV. For lithium as target material, this means that a layer of a few tens to hundreds µm suffices to reduce the energy of the proton beam to the 1.889 MeV threshold value. The remainder of the proton beam however has to be stopped by the target, so that it does not reach the patient. Therefore, in the known practice a thick lithium target is used, in which the proton beam is fully stopped.

Proton beams of 10-20 mA, 1.9-3 MeV have a power of 19-60 kW, which is fully received by the target. The target thus experiences a very high heat load which has to be dissipated in order to avoid melting and/or evaporation of the lithium target (lithium has a melting point at 180°C). Therefore, the target is usually provided with a copper backing layer for the lithium layer in which cooling channels are provided. Copper is used because of its excellent thermal conductivity (400 W/m.K).

The use of thick lithium targets comes with a number of drawbacks. Firstly, the irradiation of lithium by a proton beam may change the target physical properties. Lithium, with a moderate thermal conductivity (80 W/m.K), absorbs the protons by formation of lithium hydride having a very low thermal conductivity (5.5 W/m.K). Secondly, the nonelastic scattering of protons on the Li nuclei leads to a significant gamma flux of 0.447 MeV energy with a total flux which is comparable with the flux of neutrons.

The accompanying generation of gamma radiation is significantly reduced by the use of a thin lithium target. In that case, the stopping of the protons will occur in the backing layer. The stopping of the protons may cause blistering. In the present context, the phenomenon of blistering concerns the accumulation of hydrogen below the surface of a metal, causing it to swell and form blisters. A first effect of blistering is that hydrogen gas bubbles within the backing layer material interrupt the thermal flux through the plate, thereby producing a rise of temperature. As a consequence, the rise of temperature accelerates lithium target evaporation, reducing its lifetime. Further, heaping up of hydrogen will let the blister grow until the thin metal shell of the blister cracks, leaving a crater behind.

The hydrogen in the blisters originates from the incident protons. When a material is irradiated with a proton beam, some atoms in the material lattice structure will be displaced due to an incident proton on the atom. This causes a void in the lattice structure, which may be filled with other atoms resulting from the incident beam (i.e. hydrogen). For materials showing a high diffusivity and solubility for hydrogen, the formed hydrogen atoms diffuse or react with the atoms of the lattice. For the case of lithium, Li reacts with H to form lithium hydride and its solubility in the Li lattice is high. However, for materials showing a rather low hydrogen diffusivity and solubility, the hydrogen heaps up, preferably filling the vacancies within the lattice structure. This leads to a pressure rise in the vacancies and eventually to the formation of a blister. When the pressure within the blisters grows too high, the blister heads open and crack - either centrally with cracks running across the blister, or around the periphery. Even though copper features a very high thermal conductivity, it has a very poor resistance against blistering.

Blistering, evaporation of lithium and formation of lithium hydride affect the target lifetime negatively. The manufacturing and substitution cost of a new target aside, the disposal cost of a used target is non-negligible too. The ⁷Be is a radioactive isotope with a half-life of 53 days. As the lithium layer of the target comprises this isotope originating from the nuclear reaction upon proton bombardment, before being disposed of, the target has to be kept in an appropriate container for a period of about two years.

An example of an accelerator-based BNCT facility is disclosed by C.N. Culbertson et al. in "Inphantom characterisation studies at the Birmingham Accelerator-Generated epithermal Neutron Source BNCT facility", Applied Radiation and Isotopes, 61 (2004), pp. 733-738. The facility comprises a horizontally aligned solid thick natural lithium target on a cooled copper backing plate and a Fluental moderator. The beam shaping assembly further comprises a graphite reflector around the moderator and a Li-polyethylene sheet to provide a delimited neutron beam port. The proton beam used in this facility is a low current beam, of only 1 mA. A higher current would produce the evaporation of this target. Therefore, in this accelerator-based BNCT facility, a very large amount of time is required for every treatment (around 20 hours for a typical treatment).

A number of target designs have tried to tackle the problem of target heat removal. Patent documents US 4582667 and US 5392319 disclose annular targets mounted at the periphery of a rotary wheel. The wheel and/or the annular target comprise cooling channels through which a coolant liquid can be forced to flow. The wheel rotates while the proton beam only irradiates part of the target, providing an additional cooling mechanism. Nevertheless, such systems, which have a very high mechanical complexity, do not provide any suitable solution for the appearance of blistering phenomena and therefore for increasing the lifetime of the target. Finally, such facilities require enormous costs and overlong treatment times.

### Aims of the Invention

The present invention aims to provide a device for generating neutrons in an accelerator-based BNCT facility, the device comprising a lithium target for the generation of neutrons which overcomes the drawbacks of the prior art.

Particularly, the present invention aims to provide a device for generating neutrons which does not suffer from the blistering phenomenon, and which allows sufficient heat removal to avoid melting of said target.

Another aim of the present invention is to provide a device for generating neutrons which drastically reduces the overlong treatment times required in the state of the art BNCT facilities.

Further, it is an aim of this invention to provide a neutron generating device which is less costly than the state of the art devices.

### Summary of the Invention

The present invention is related to a device, as set out in the appended claims, for generating neutrons out of an incident beam of protons and suitable for neutron capture therapy.

According to one aspect of the invention there is provided a device for generating neutrons from a low energy, high current proton beam, the device comprising a target for said proton beam, the target comprising one or more panels. Each of said panels comprises a backing layer and a thin layer of lithium provided on said backing layer. Said device is characterised in that the backing layer is made of a material having as one of its main constituents an element chosen out of the group comprising Fe, Ta and V. By low energy proton beam, we understand a proton beam having energies above the reaction threshold (i.e. 1.889 MeV) and below 3 MeV. By high current proton beam, we understand a current sufficient for providing a neutron flux allowing treating a patient in no more than 30 minutes. This can be achieved with a proton beam having a current intensity equal to or higher than 20 mA.

Preferably, in said target, the one or more panels are inclined with respect to the incident proton beam.

Preferably, in said target, the thickness of the layer of lithium is determined such that the proton beam exits said layer of lithium with an energy not higher than 1.889 MeV.

More preferably, the panels comprise means for cooling the panel.

The layer of lithium is preferably under metallic form, i.e. not under hydrided, oxided, or nitrided form. Preferably, the panels each comprise a protective layer. Said protective layer is advantageously deposited on top of the lithium layer. Thereby, the chemically reactive lithium is protected from oxidation.

According to an even more preferred embodiment of the invention, the target comprises two panels, arranged under an angle with respect to each other, said angle being larger than zero and smaller than 180°. Preferably, said angle is 60°. Using this structure, the proton beam can be scanned over a large area, thereby distributing the heath generation over a larger surface.

According to a more preferred embodiment of the invention, the device for generating neutrons further comprises a beam shaping assembly. The beam shaping assembly comprises a moderator. The moderator is preferably made of magnesium fluoride (MgF₂). Preferably, the length of the moderator is determined as the smallest length leading to a dose at skin level (Dₜᵢₛₛᵤₑ(0 cm)) lower than or equal to 8 Gy-eq.

According to a second aspect of the invention, there is provided a neutron generating apparatus comprising: an accelerator for producing a low energy, high current proton beam (e.g. 20 mA, 1.9 to 3 MeV), a beam transport system comprising magnets, a scanner system and a collimator. The neutron generating apparatus according to this aspect of the invention is characterised in that it further comprises: a device for generating neutrons according to the invention. Preferably, in said neutron generating apparatus, the device for generating neutrons is provided at an outlet of the collimator.

According to a third aspect of the invention, there is provided a method for generating neutrons from a low energy, high current proton beam. The method is characterised in that it comprises the steps of: providing a backing layer made of a material having as one of its main constituents an element chosen out of the group comprising Fe, Ta and V; providing a layer of lithium on said backing layer and irradiating said layer of lithium with said proton beam. Preferably, the backing layer is cooled. One may preferably deposit a protective layer onto the layer of lithium.

According to a fourth aspect of the invention, there is provided a use of the device for generating neutrons according to the invention for boron neutron capture therapy.

According to a last aspect of the invention, there is provided a therapeutic method of treating cancer such as gliomas, melanomas and head, neck and liver cancers, the method comprising the step of treating a patient with boron neutron capture therapy, wherein the neutrons are generated by the device for generating neutrons of the invention.

### Brief Description of the Drawings

Figure 1 represents a view of the collimator outlet and the target assembly according to the invention.

Figure 2 represents the assembly of a target panel.

Figure 3 represents a cross section of the lithium layer, the backing layer and the main body of a target panel.

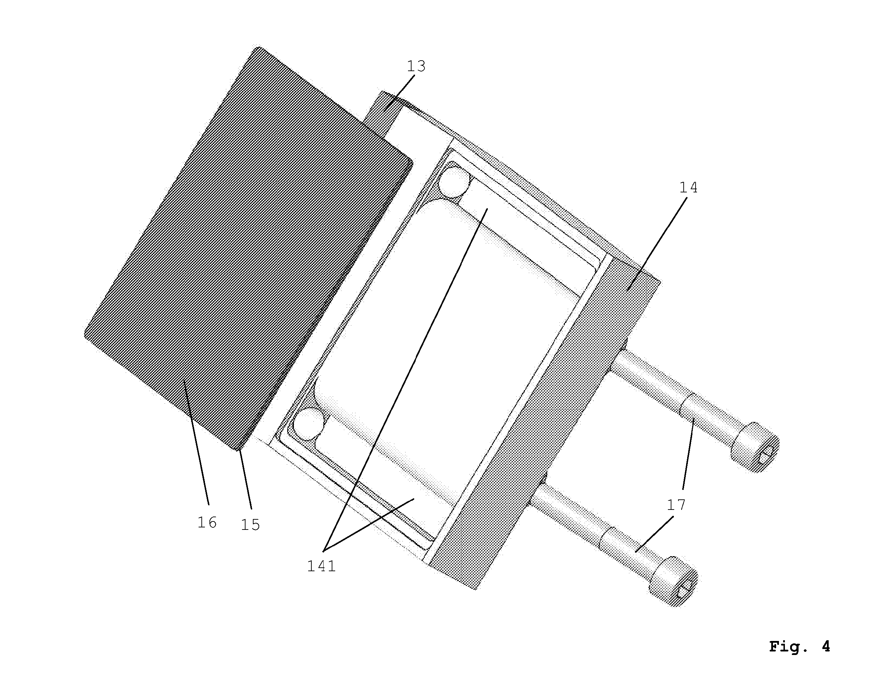

Figure 4 represents the assembly of figure 2 viewed from the opposite side.

Figure 5 represents a cross section of the collimator and target assembly.

Figure 6 represents the beam shaping assembly.

Figure 7 shows the correlation between the maximal thermal neutron flux and the dose produced at skin level for MgF₂ and Fluental as moderator materials.

Figure 8 represents neutron energy spectra obtained by using MgF₂ and Fluental as moderator materials.

Figure 9 shows the correlation between the maximal thermal neutron flux and the dose produced at skin level for MgF₂ as moderator material, with different densities.

Figure 10 shows the evolution of the dose at skin level as a function of the moderator length for two moderator diameters and MgF₂ as moderator material.

### Detailed Description of the Invention

In a thick lithium target, almost all the protons coming from the irradiating proton beam are stopped within the lithium layer. The backing layer in this case hardly receives any incoming proton, so the problem of blistering does not subsist for the copper backing. On the other hand, when the lithium target is made thin, part of the irradiating protons are stopped in the backing layer. The material of the backing layer should preferably show a high resistance against blistering in order to extend the target lifetime as much as possible. Any blistering of the backing layer will crack also the thin lithium layer.

Though, thin lithium targets are preferred over thick ones, due to a decreased generation of unwanted gamma radiation. Thin lithium targets may also potentially have a longer lifetime than their thick counterparts, provided that the backing layer is made of a material showing a high resistance against blistering, and that a sufficient amount of heat is removed. The use of copper as backing layer is not recommended, due to the occurrence of blistering.

It has been shown by research carried out by the inventors that the backing layer of a thin lithium target is preferably made of a material comprising iron (Fe), tantalum (Ta) or vanadium (V). These three materials show a high resistance against blistering. Furthermore, as the final proton absorption will occur in the backing layer, the backing layer material should be chosen such that the proton scattering does not cause gamma emission. Broadly speaking, the latter condition is satisfied for all nuclei heavier than A1. This condition is also satisfied for Fe, Ta and V. Compared to Ta and V, Fe is a preferred choice of backing layer material due to its low cost and machining ease.

However, the thermal conductivity of Fe (80 W/m.K), Ta (57 W/m.K) and V (30 W/m.K) is much lower than the thermal conductivity of Cu. The solution brought by the present invention to overcome the heat dissipation problem is threefold. Firstly, a very efficient cooling circuit has to be provided in order to keep the temperature of the lithium layer below its melting point of 180°C. Secondly, in order to cut the heat load per unit area on the target, the target of the present invention is inclined with reference to the incident beam's axis. As a final point, the incident proton beam is magnetically scanned on the target using a scanning pattern in order to distribute the heat load as much as possible over the entire useful target area.

A particular embodiment of the present invention is described as follows. The target of this embodiment may be used in an accelerator-based BNCT facility. There is thus provided an accelerator producing a proton beam and a high-energy beam transport line carrying the beam to a collimator. The proton beam is a 20 mA, 2.5-3 MeV beam and may have a diameter of about 16 mm at the exit of the collimator. At the exit of the collimator a target is provided, which is arranged for being irradiated by the proton beam and hence arranged for generating neutrons.

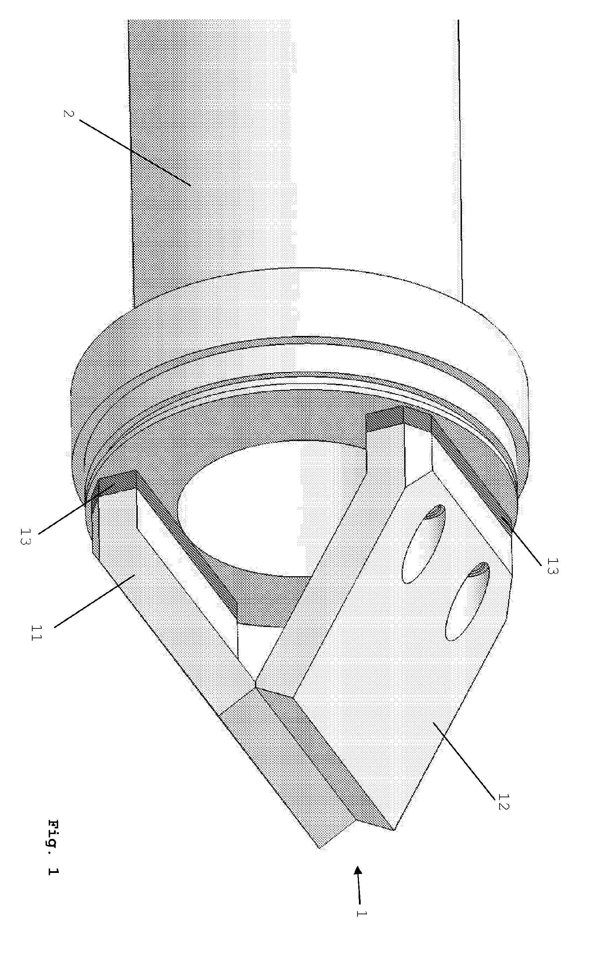

According to the latter embodiment of the invention, the target 1, as depicted in figure 1, is provided at the outlet of a tubular collimator 2. The target 1 comprises two panels, 11 and 12 mounted at an angle of approximately 30° with reference to the symmetry axis of the collimator. A layer 13 of electrically insulating material is interposed between each of the panels 11, 12 and the collimator 2. Hence, the panels are mounted so as to be electrically isolated from the collimator, which is needed for current measurement.

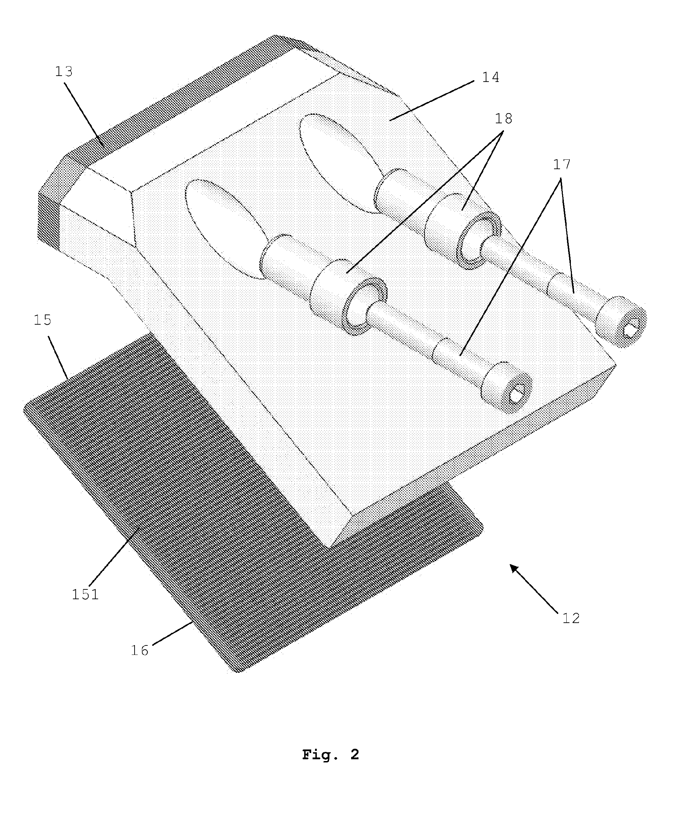

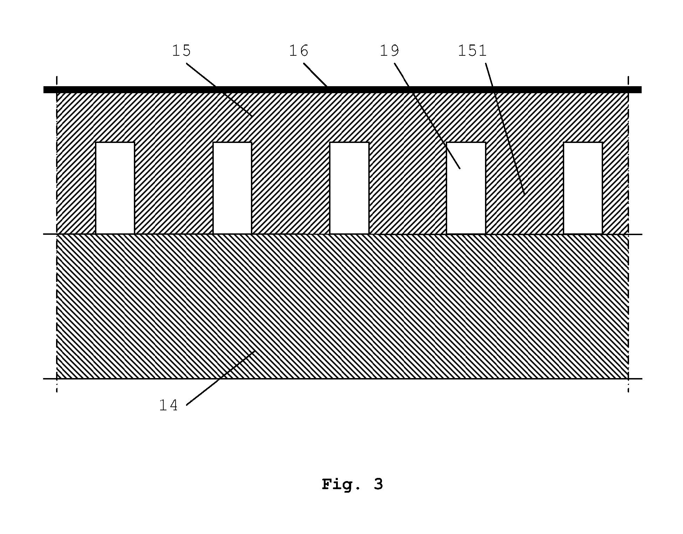

Referring to figure 2, each panel 11, 12 comprises a main body 14, a backing layer 15 and a layer 16 of lithium. The layer 16 of lithium is provided onto the backing layer 15. At the opposite side of the lithium layer, the backing layer 15 is provided with fins 151 (see also figure 3). The finned backing layer 15 is arranged to be assembled onto the main body 14, with the fins 151 facing the main body.

Referring to figure 3, when the finned backing layer 15 and main body 14 are assembled, the space between the fins 151 constitute cooling channels 19 through which a cooling liquid is forced to flow. The main body is provided with inlet and outlet manifolds 141. The inlet manifold distributes the cooling liquid equally over all cooling channels 19 and the outlet manifold collects the cooling liquid. The collimator 2 is equipped with dedicated inlet and outlet ports for supplying and collecting the cooling liquid for the target. In the present embodiment, the inlet and outlet manifolds are symmetrical counterparts. Their role may thus be inverted.

Each panel 11, 12 is fixed to the collimator 2 through bolts 17 (figure 2). In order to ensure the electrical insulation of each panel, insulators 18 are provided between the panel and the bolts.

The panels 11, 12 are mounted at the outlet of the collimator 2 with the lithium layer 16 facing the incident proton beam. The lithium layer has a thickness sufficient to reduce the energy of the incident proton beam to less than or equal to 1.8 MeV. Below this energy, the beam is not useful for generating neutrons. For a 2.8 MeV proton beam, this results in a beam path length through lithium of about 110 µm. As the layer of lithium is 30° inclined with reference to the beam's axis, the necessary thickness of the lithium layer to achieve the above beam path length is 55 µm.

The layer of lithium should be under metallic form, i.e. not under hydrided, oxided, or nitrided form. As lithium is a reactive element, it is preferably coated with a thin protective layer to avoid contact with air during installation. Lithium is highly chemically reactive and reacts readily with water and air. The material and thickness of the protective layer are selected so as to provide a good chemical protection, and for minimizing proton beam loss and heat generation. A layer of 2 µm of stainless steel has been determined to achieve these goals. The protection of the lithium material can also be improved by depositing successive layers of lithium, each protected by a layer of protective material, e.g. 10 µm of lithium, then 2 µm of stainless steel, repeated five times.

The finned backing layer 15 and the main body 14 are preferably made out of a carbon steel with a low carbon content. The carbon content of the steel for the backing layer and main body lies preferably below 0.8%. The main body 14 may be made out of a different material than the backing layer 15, e.g. a material having high thermal conductivity such as copper (Cu) or silver (Ag). The finned backing layer 15 may also comprise two layers : a first layer, in contact with the lithium layer, made of a blistering resistant material (Fe, Ta or V) of a thickness sufficient for stopping all remaining protons, and a second layer made of a material having high thermal conductivity such as copper (Cu) or silver (Ag).

The manufacture and assembly of the target proceeds according to the following steps. For each of the two panels, a main body 14 and finned backing layer 15 are manufactured. In the main body 14, the inlet and outlet manifolds 141 are machined, and a recess is formed for housing the backing layer 15. The backing layer 15 is provided with fins 151 on one side. On the other side, a layer 16 of lithium is deposited on the plate's surface. The deposition of lithium on the backing layer 15 may be performed by physical vapour deposition or any other suitable technique. A protective layer may be deposited onto the lithium layer 16 afterwards. Also, a succession of lithium and protective layers may be deposited.

The backing layer 15 is subsequently assembled into the recess provided in the main body with the fins facing the main body. Next, the backing layer is bonded to the main body. Backing layer 15 and main body 14 may be bonded by brazing, laser welding, diffusion bonding or any other suitable technique. The bonding between backing layer and main body provides the necessary sealing for the cooling channels 19. The bond must be strong enough to withstand the pressure of the cooling liquid flowing through the channels 19.

An electrical insulation layer 13 is provided at the base of each panel 11 and 12. Finally, the two panels 11 and 12 are mounted on the collimator 2 such that the lithium layers are oriented at a 30° angle with reference to the symmetry axis of the collimator. The panels are preferably fixed to the collimator 2 by bolts 17.

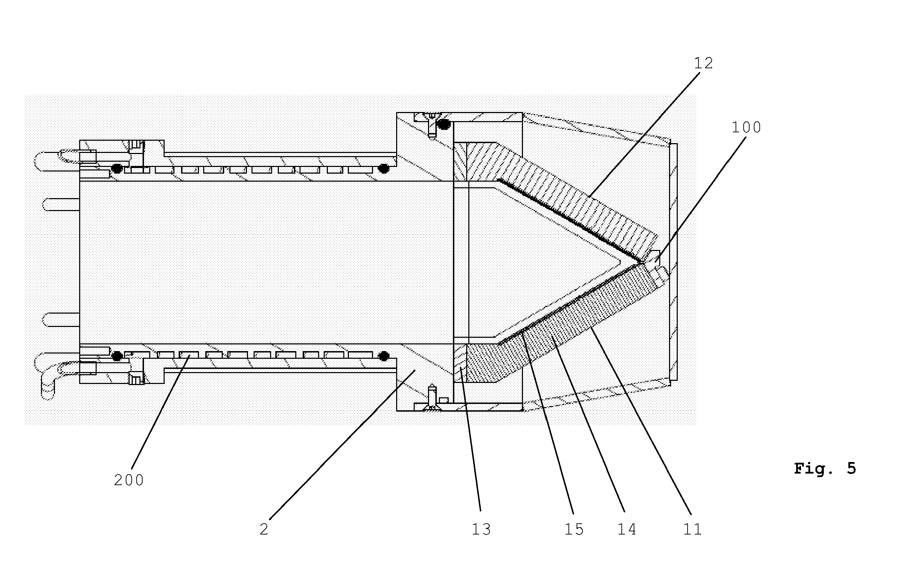

Figure 5 shows a cross section of the collimator 2 and target assembly according to the present embodiment. At the top of the target 1, where the two panels 11 and 12 join, a proton stopper 100 is provided. The collimator 2 is provided with cooling channels 200. The inlet and outlet ports for providing cooling liquid from the collimator to the panels 11 and 12 are not shown on figure 5.

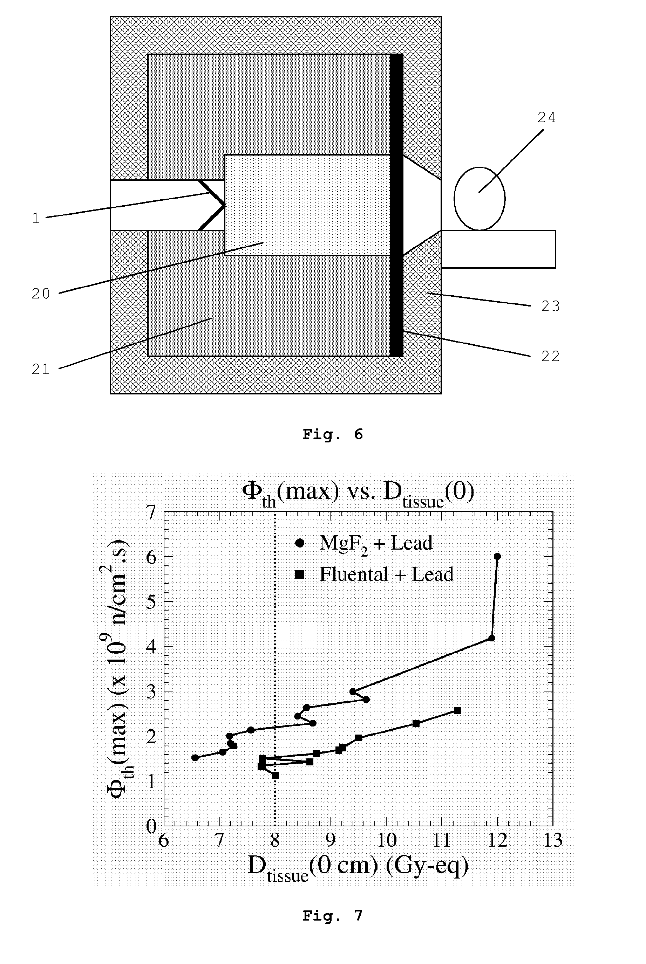

A moderator assembly finally tailors the neutron flux produced by the target. The moderator assembly - also called a beam shaping assembly - has to provide an epithermal neutron beam with the specified characteristics. As shown in figure 6, the preferred moderator assembly for the target of the present invention comprises a cylindrical moderator 20 arranged directly after the target. Around the moderator 20 a reflector 21 is provided. At the end of the moderator 20 and reflector 21, opposite to the target, a filter 22 is provided. A delimiter 23 is provided around the reflector 21 and the filter 22. The patient 24 is placed at the exit of the beam shaping assembly, in front of the moderator 20.

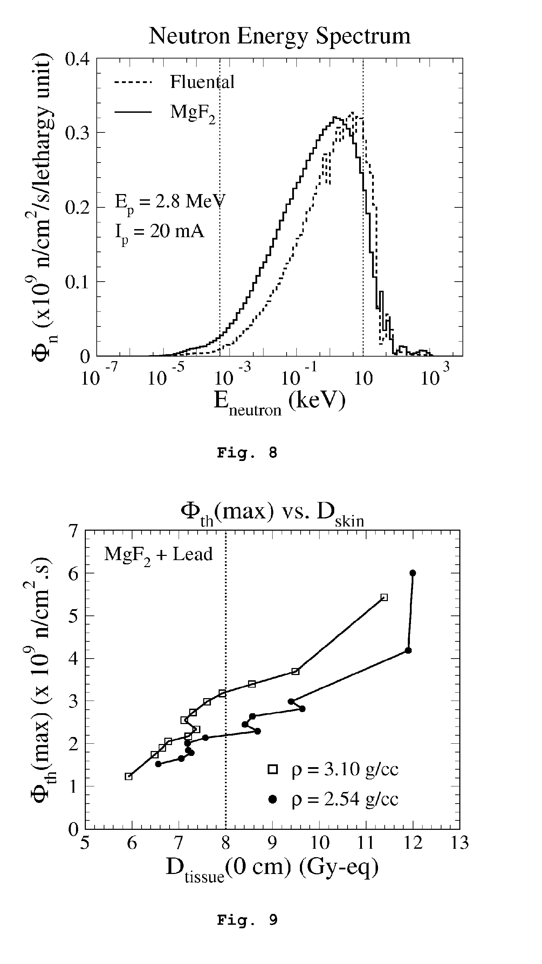

Various materials can be used as moderators, such as D₂O, Al₂O₃, Fluental® and MgF₂. Research carried out by the inventors shows that magnesium fluoride (MgF₂) as moderator material displays a clear advantage compared to Fluental. This is shown in the comparative graphs of figures 7 and 8. The values shown in the graphs are computed values obtained with a Monte Carlo simulation code MCNPX. In this simulation, the radiation dose deposited in the brain tissues is computed by summing the various components from neutrons and photons. For the components due to neutron interactions, fluence-to-kerma conversion factors are taken from the ENDF/B-VI library. For the photon dose, mass energy absorption coefficients from ICRU46 are used.

The total effective dose to either tumour or healthy tissue is calculated in Gray-equivalent (Gy-eq) by using RBE values of 3.2 for hydrogen and nitrogen doses, 1 for photon dose, 1.3 and 3.8 for ¹⁰B dose in healthy tissue and tumour, respectively. The ¹⁰B concentrations in healthy tissue and tumour are set to 12 ppm and 48 ppm, respectively. Referring to figure 7, each point in the graph corresponds to a different moderator length and shows the values obtained for the maximal thermal flux (Φₜₕ(max)) and dose at skin level (Dₜᵢₛₛᵤₑ(0 cm)) parameters. These two parameters are clearly correlated and both decrease with the moderator length. MgF₂ displays a clear advantage when compared to Fluental as the thermal flux remains 40% larger when the target dose of Dₜᵢₛₛᵤₑ(0 cm)=8 Gy-eq is reached. The optimal moderator length may be chosen as the smallest length leading to Dₜᵢₛₛᵤₑ(0 cm) <8 Gy-eq: it is equal to 35 cm for MgF₂ compared to 45 cm for Fluental. In figure 7, lead is taken as reflector material.

Figure 8 compares the neutron energy spectra obtained with the two optimal moderators at the beam shaping assembly exit: the MgF₂ spectrum is clearly softer than Fluental, with a fast neutron component (En > 10 keV) representing only 6% of the total flux compared to 8% for Fluental. In "Optimization of an accelerator-based epithermal neutron source for neutron capture therapy", (Appl. Radiat. Isot. 61(5), 2004, pp. 1009-1013), Kononov et al. investigate the performance of MgF₂ and Fluental moderator materials using thick lithium targets and also conclude that MgF₂ performs better in obtaining an epithermal neutron beam compared with Fluental.

Results presented in figure 7 correspond to a MgF₂ density of 2.54 g/cm³. This density is a critical parameter as demonstrated in figure 9. For a density of 3.1 g/cm³ (corresponding to the maximal density of a MgF₂ crystal), the optimal length is reduced to 27 cm and the corresponding maximal thermal flux is increased to 3.2 10⁹ n / cm².s.

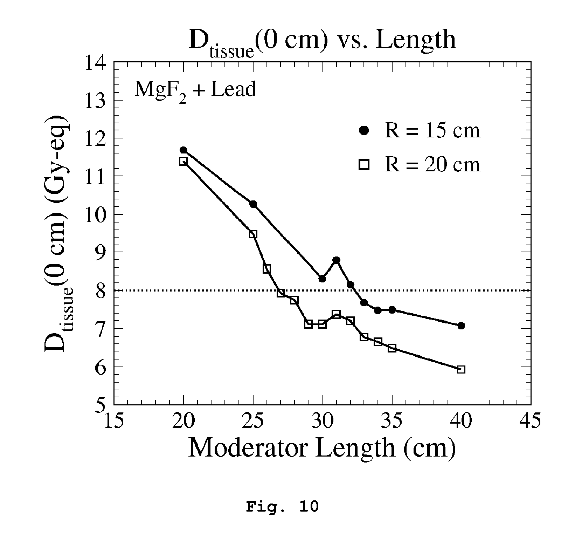

Another critical parameter for the beam shaping assembly is the moderator radius. As shown in figure 10 for MgF₂ (density of 3.1 g/cm3), the moderator optimal length is significantly smaller when the moderator radius is increased from 15 cm to 20 cm. This length reduction is directly translated into a thermal flux increase of about 50%. The increase in thermal flux for radius values beyond 20 cm becomes minor.

The reflector surrounding the moderator can be made out of graphite, Al₂O₃ or lead. Considering a radial thickness of 30 cm for this reflector, graphite and lead result in similar dose-depth profiles in healthy tissue and tumour but for graphite the thermal flux is reduced by as much as 25%. Despite its heavy weight, lead is thus a better choice for the reflector. It can also serve as a very effective shielding element against the radiation emitted by the activated lithium target. The radial thickness of the reflector has some influence on the produced neutron flux but the gain in flux becomes minor for values beyond 30 cm.

A delimiter, comprising a neutron-capturing element, preferably surrounds the reflector. The choice of a neutron-capturing element in the delimiter and its concentration is an additional element affecting the performance of the beam shaping assembly. Boron-Polyethylene is not an acceptable choice as delimiter material, because of the γ-rays emitted by the neutron capture on Boron. These γ-rays affect the dose-depth profile in healthy tissue, preventing the reaching of a Dₜᵢₛₛᵤₑ(0 cm) value below 8 Gy-eq. The use of lithiated polyethylene containing at least 3% of enriched ⁶Li provides the best results but, due to the large surface covered by the delimiter 23, reveals not to be economically feasible. The delimiter 23 in the present embodiment is preferably made out of lithiated polyethylene with about 7.5% of natural lithium, which represents the best compromise in terms of efficiency and cost.

### Description of a Preferred Embodiment of the Invention

According to a preferred embodiment, the backing layer 15 has dimensions of 10 cm x 10 cm. The fins provided on the backing layer 15 have a pitch of 1.5 mm, are 1 mm high and 1 mm thick. The spacing between the fins is thus 0.5 mm. The thickness of the backing layer, without the fins, is 0.5 mm. A total of 68 fins are provided on the backing layer. The lithium layer has a thickness of 55 µm.

The target is designed to be irradiated with a 2.8 - 3 MeV, 20 mA proton beam. The incident proton beam on the target is collimated by a collimator so as to have a diameter of about 16 mm. In order to distribute the heat load over the entire target, a magnetic scanner is provided upstream of the collimator to scan the beam on the target according to a predetermined pattern. As such the target receives a peak heat load of 450 W/cm². In order to withdraw the heat, water is used as cooling liquid and a flow rate per panel of about 20 litres/minute should be maintained. The target has an estimated lifetime of 80 beam hours.

The moderator is preferably made of MgF₂, with a density of at least 2.54 g/cm³. It has a cylindrical shape, of 20 cm radius and its length is adjusted in function of the MgF₂ density in order to reach a maximal dose value of 8 Gy-eq at skin level in healthy tissues.

The reflector is preferably made of lead with a radial thickness of 30 cm. The delimiter is preferably made of lithiated polyethylene with about 7.5% lithium. The whole beam shaping assembly should provide an epithermal neutron beam, resulting into a thermal neutron flux of 2.10⁹ n/cm².s at a depth of 2.5 cm in tissue along the neutron beam centreline.

## Claims

1. A device for generating neutrons from a low energy, high current proton beam, the device comprising a target (1) for said proton beam, the target comprising one or more panels (11, 12), each of said panels comprising a backing layer (15) and a thin layer (16) of lithium provided on said backing layer, **characterised in that** said backing layer (15) is made of a material having as one of its main constituents an element chosen out of the group comprising Fe, Ta and V.

2. The device according to claim 1, wherein said target is arranged in such a way that said one or more panels (11, 12) are inclined with respect to said proton beam.

3. The device according to claim 1 or 2, wherein the thickness of said layer (16) of lithium is determined such that the proton beam exits said layer of lithium with energy not higher than 1.889 MeV.

4. The device according to any one of the preceding claims, wherein one or more of said panels comprise a protective layer on top of the layer of lithium for protecting the lithium layer from oxidation.

5. The device according to any one of the preceding claims, wherein one or more of said panels (11, 12) comprises means (19, 151) for cooling the panel.

6. The device according to any one of the preceding claims, wherein said target (1) comprises two panels (11,12) arranged under an angle with respect to each other, said angle being larger than zero and smaller than 180°.

7. The device according to claim 6, wherein said angle is 60°.

8. The device according to any one of the preceding claims, **characterised in that** it further comprises a beam shaping assembly comprising a moderator (20) .

9. The device according to claim 8, wherein the moderator (20) is made of magnesium fluoride (MgF₂) .

10. The device according to claims 8 or 9, wherein the length of the moderator (20) is determined as the smallest length leading to a dose at skin level lower than or equal to 8 Gy-eq.

11. A method of generating neutrons from a low energy, high current proton beam, **characterised in that** the method comprises the steps of:
- providing a backing layer (15) made of a material having as one of its main constituents an element chosen out of the group comprising Fe, Ta and V.
- providing a layer (16) of lithium on said backing layer, and
- irradiating said layer of lithium with said proton beam.

12. The method according to claim 11, further comprising the step of cooling said backing layer (15).

13. The method according to claim 11 or 12, further comprising the step of depositing a protective layer onto the layer of lithium.

14. Use of the device according to any one of the claims 1 to 10 for boron neutron capture therapy.
